# EUROPEAN PATENT SPECIFICATION

(11) **EP 2 811 845 B1**
(45) Date of publication and mention of the grant of the patent: **09.12.2015**
(21) Application number: 12703533.5
(22) Date of filing: 10.02.2012
(51) Int. Cl.: A23C 9/20

(54) **MATERNAL SUPPLEMENT TO ENHANCE IMMUNE SYSTEM OF AN INFANT**
MÜTTERLICHES ERGÄNZUNGSMITTEL ZUR STÄRKUNG DES IMMUNSYSTEMS EINES SÄUGLINGS
SUPPLÉMENT POUR MÈRES VISANT À RENFORCER LE SYSTÈME IMMUNITAIRE D'UN NOURRISSON

(43) Date of publication of application: 17.12.2014
(60) Divisional application: 15187545.7
(73) Proprietor: N.V. Nutricia, 2712 HM Zoetermeer (NL)
(72) Inventor: GARSSEN, Johan, NL-3433 DA Nieuwegein (NL); HOGENKAMP, Astrid, NL-3552 EK Utrecht (NL); VAN ESCH, Elisabeth Catharina Adriana Maria, NL-3527 WH Utrecht (NL); KNIPPELS, Leon Matthieu Johannes, NL-3523 VJ Utrecht (NL)
(74) Representative: Bailey, Jennifer Ann
(86) International application number: PCT/EP2012/052299
(87) International publication number: WO 2013/117235

(56) References cited:
- WO-A1-2011/008086
- WO-A2-2005/039597
- JOSE STAM ET AL: "A mixture of three prebiotics does not affect vaccine specific antibody responses in healthy term infants in the first year of life", VACCINE, ELSEVIER LTD, GB, vol. 29, no. 44, 25 July 2011 (2011-07-25) , pages 7766-7772, XP028309438, ISSN: 0264-410X, DOI: 10.1016/J.VACCINE.2011.07.110 [retrieved on 2011-07-29]
- VOS ET AL: "Dietary supplementation with specific oligosaccharide mixtures decreases parameters of allergic asthma in mice", INTERNATIONAL IMMUNOPHARMACOLOGY, ELSEVIER, AMSTERDAM, NL, vol. 7, no. 12, 3 October 2007 (2007-10-03), pages 1582-1587, XP022284914, ISSN: 1567-5769, DOI: 10.1016/J.INTIMP.2007.07.024
- VOS A P ET AL: "Dietary supplementation of neutral and acidic oligosaccharides enhances Th1-dependent vaccination responses in mice", PEDIATRIC ALLERGY AND IMMUNOLOGY, COPENHAGEN, DK, vol. 18, no. 4, 1 June 2007 (2007-06-01), pages 304-312, XP002520520, ISSN: 0905-6157, DOI: 10.1111/J.1399-3038.2006.00515.X

## Description

### FIELD OF THE INVENTION

The present invention relates generally to a composition administered to a mother who is breast-feeding her infant for enhancing an immune system and/or preventing an immune system related disorder in the infant.

### BACKGROUND TO THE INVENTION

During the first weeks or months of life, a number of external factors may influence the infant's growth and development. In particular, on-going research is making it more and more apparent that the diet of the mother while breast-feeding her infant can affect the infant's health, both immediately and later in life.

It is therefore desirable to provide maternal supplements which assist the mother in providing her child with health benefits, including immune-related benefits such as a decrease in allergic reactions, including long-term benefits to the infant later in life when the infant is no longer breast-fed.

### SUMMARY OF THE INVENTION

The present invention is based on the discovery made by the inventors that maternal supplementation with a non-digestible oligosaccharide while an offspring is receiving breast milk has a beneficial programming effect on the immune response of the offspring.

Hence, the present invention relates to a maternal composition for a lactating postpartum female, the composition comprising a non-digestible oligosaccharide for enhancing the immune system of an infant being breast-fed by the female. This composition can be advantageously used for the enhancement of the immune system in the infant wherein the composition is administered to the mother of the infant during lactation postpartum and the advantageous effect in the infant can be observed later in life, e.g. during childhood and/or adolescence. The enhancement of the immune system may include an improvement (e.g. a decrease) in an allergic reaction.

Thus, according to a first aspect, the present invention provides a composition comprising a non-digestible oligosaccharide for use in enhancing an immune system of a mammalian subject, wherein the composition is for administration to a lactating postpartum female who is breast-feeding the mammalian subject.

According to another aspect, the present invention provides a use of a non-digestible oligosaccharide for the preparation of a composition for enhancing an immune system and/or preventing an immune system related disorder in a mammalian subject, wherein the composition is for administration to a lactating and postpartum female who is breast-feeding the mammalian subject.

According to a further aspect, the present invention provides a method of enhancing and/or preventing an immune system related disorder in a mammalian subject, the method comprising providing or administering a composition comprising a non-digestible oligosaccharide to a lactating and postpartum female who is breast-feeding the mammalian subject.

According to yet another aspect, the present invention provides a method of providing or administering a composition comprising a non-digestible oligosaccharide to a lactating, postpartum female who is breast-feeding a mammalian subject, wherein the composition enhances an immune system and/or prevents an immune system related disorder in the mammalian subject.

As will be apparent from the following description, suitable embodiments of the present invention provide for use of a composition comprising a non-digestible oligosaccharide for preventing an allergic reaction in the mammalian subject. The immune-related benefits such as prevention of an allergic reaction may include long-term benefits such as prevention of an allergic reaction later in life when the infant is no longer breast-fed.

### BRIEF DESCRIPTION OF THE FIGURES

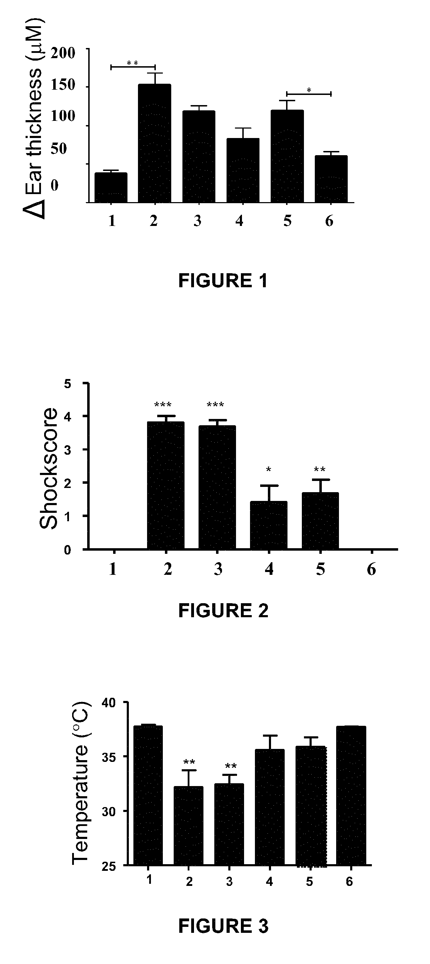

Figure 1 is a bar graph showing the change in ear thickness of mice after ovalbumin (OVA) challenge in an acute allergic skin response test as described in Example 1. Mice were challenged with 20 µg/20µl OVA intradermally and the ear swelling as a result of the acute allergic skin response was measured after one hour. The numbers across the *x*-axis represent the group of mice (as set out in Table 1) based on the treatment and diet of the mother, and the values along the *y*-axis represent the change (µM) in ear thickness measured in the offspring (* P<0.05, ** P<0.01, **** P<0.00001).
Figure 2 is a bar graph showing the shock score observed in mice after OVA challenge in an acute allergic skin response test as described in Example 1. Mice were challenged with 20 µg/20µl OVA intradermally and shock score was assessed 30 minutes after challenge. The numbers across the x-axis represent the group of mice (as set out in Table 1) based on the treatment and diet of the mother, and the values along the *y*-axis represent the shock scores measured in the offspring (1: puffy eyes, 2: shortness of breath, piloerection, 3: impaired mobility, 4: no mobility, 5: death) (* P<0.05, ** P<0.01, *** P<0.001 = significantly different from group 1 of Table 1 (i.e. control)).
Figure 3 is a bar graph representing average temperature of mice after OVA challenge in an acute allergic skin response test as described in Example 1. Mice were challenged with 20 µg/20µl OVA intradermally and temperature was taken 30 minutes after challenge. The numbers across the *x*-axis represent the group of mice (as set out in Table 1) based on the treatment and diet of the mother, and the values along the *y*-axis represent the temperature of the mice 30 minutes after challenge. (* P<0.05, ** P<0.01, *** P<0.001 = significantly different from the mice in group 1 of Table 1 (i.e. control)).

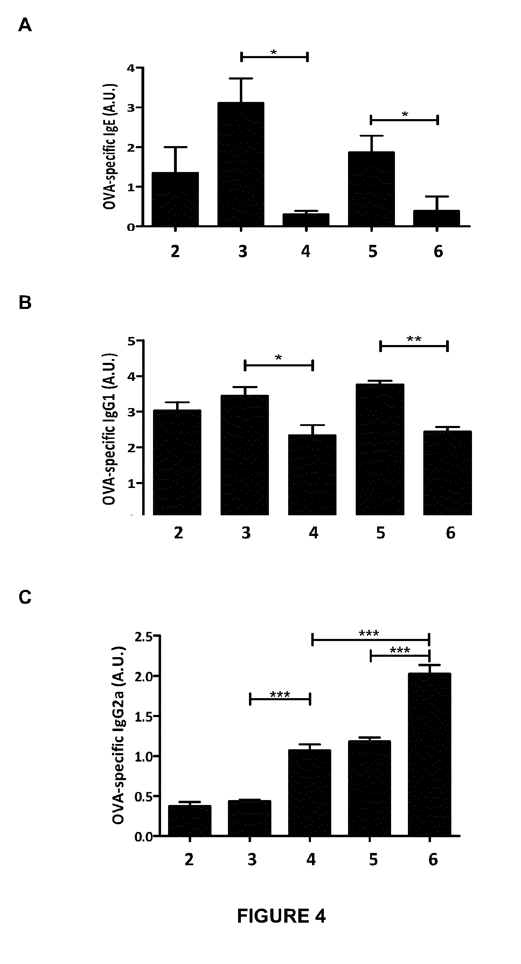

Figure 4 contains three bar graphs showing the level of OVA-specific immunoglobulin (IgE, IgG1, IgG2a) in mice after an acute allergic skin response test as described in Example 1. OVA-specific Ig-levels were measured by ELISA. The numbers across the *x*-axis represent the group of mice (as set out in Table 1) based on the treatment and diet of the mother, and the values along the *y*-axis represent levels of IgE (Figure 4A), IgG1 (Figure 4B), and IgG2a (Figure 4C).

### DETAILED DESCRIPTION OF THE INVENTION.

Bibliographic references mentioned in the present specification are for convenience listed in the form of a list of references and added at the end of the Examples. The whole content of such bibliographic references is herein incorporated by reference.

### Definitions

For convenience, certain terms and phrases employed in the specification and appended claims are collected here.

Reference to an element by the indefinite article "a" or "an" does not exclude the possibility that more than one of the element is present, unless the context clearly requires that there be one and only one of the elements. The indefinite article "a" or "an" as used herein thus usually means "at least one".

The term "breast-feeding" is herein defined to be nursing or providing of breast-milk to an offspring.

The term "comprising" and its conjugations is used in its non-limiting sense to mean that items following the word are included, but items not specifically mentioned are not excluded. Accordingly, the term "comprising" encompasses the more restrictive terms "consisting essentially of" and "consisting of."

The phrase "degree of methoxylation" (also referred to as DE or "degree of esterification") is intended to mean the extent to which free carboxylic acid groups been esterified (e.g. by methylation). For example, with reference to pectin, the phrase "degree of methoxylation" or "DE" or "degree of esterification" refers to the extent to which free carboxylic acid groups contained in the polygalacturonic acid chain have been esterified (e.g. by methylation).

The phrase "enhancing the immune system of a mammalian subject" (and similar) is generally defined as providing or causing a beneficial improvement in the immune system of the mammalian subject, including but not limited to strengthening, improving, and/or stimulating the immune system of the mammalian subject. The strengthening, improvement, and/or stimulation may be with respect to a control group, which may be with respect to a mammalian subject whose mother did not ingest the composition comprising the non-digestible oligosaccharide described herein during lactation.

To enhance an immune system, as is well-known in the art, means to at least increase a mammalian subject's capacity to respond to foreign or disease-specific antigens, i.e., those cells primed to attack such antigens are increased in number, activity and ability to detect and destroy antigens. In some other suitable embodiments, the enhancement of an immune system refers to preventing, protecting against and/or decreasing an unwanted immune response. Immune response may be measured by any number of standard tests known in the art, including tests performed *in vitro* on a sample collected from a mammalian subject. Enhanced immune response may also be indicated by a change (increase or decrease) of physical manifestations such as fever and inflammation, as well as healing of systemic and local infections, reduction in symptoms in disease and the like.

An illustrative example of an enhanced immune system includes an improved allergic response in the mammalian subject. Accordingly, in some specific embodiments, the present invention aims to improve the allergic response in the mammalian subject or a related disease including but not limited to atopic dermatitis, asthma, occupational asthma, food allergy (including cow's milk allergy and others), allergic rhinitis (e.g. pollen allergy), dust mite allergy and other forms of hypersensitivity like systemic anaphylaxis and acute urticaria.

The phrase "immune system related disorder" as used herein refers to unwanted, undesirable and/or excessive immune system reactions, including unwanted, undesirable or excessive allergic reactions, autoimmune conditions, and the like. These reactions may be caused in part or in total by over stimulation of the immune system that may lead to excessive inflammation and/or destruction of normal cells and/or tissues. Immune system related disorder(s) may be damaging, uncomfortable, and/or occasionally fatal. Non-limiting examples of disorders may include allergy Type 1, allergy Type 2, allergy Type 3, allergy Type 4, eczema, asthma, rhinitis, hay fever, rhino-conjunctivitis, wheezing, intestinal infections, respiratory infections, atopic dermatitis, allergic dermatitis, food allergy and the like.

The term "lactating" refers to both the act of secreting milk from the mammary glands and the period of time that a female breast-feeds its offspring. In human beings, the terms "breast-feeding", "nursing" and "lactating" may be interchangeably used.

The term "non-digestible" as used in herein refers to saccharides and/or oligosaccharides which are not digested in the intestine by the action of acids or digestive enzymes present in the human upper digestive tract (small intestine and stomach), but which are fermented by the human intestinal flora.

The term "post-partum" is herein defined to be the period beginning immediately after the birth of a child. Postpartum refers to the mother.

The phrase "preventing an immune system related disorder" (and the like) is herein defined to be a prophylactic treatment of a mammalian subject in need thereof, including but not limited to preventing, relieving, altering, reversing, affecting, inhibiting the development or progression of, ameliorating, or curing (1) an immune system related disorder, or (2) a symptom of an immune system related disorder, or (3) a predisposition toward an immune system related disorder, including conferring protective immunity on the mammalian subject.

The term "soluble" as used herein with reference to a non-digestible oligosaccharide means that the substance is soluble according to the method described by L. Prosky et al, 1988.

The term "mammalian subject" is herein defined as a mammalian individual, suitably a human. For purposes of research, the subject may be a non-human. For example the subject may be an animal suitable for use in an animal model, *e.g.,* a pig, horse, mouse, rat, cow, dog, cat, cattle, non-human primate (*e.g.* chimpanzee) and the like.

### Maternal Compositions

According to a first aspect, the present invention provides a composition comprising, consisting of, or consisting substantially of a non-digestible oligosaccharide for use in enhancing an immune system of a mammalian subject, wherein the composition is for administration to a lactating, postpartum female who is breast-feeding the mammalian subject.

According to further aspect, the present invention provides a use of a non-digestible oligosaccharide for the preparation of a composition for enhancing an immune system and/or preventing an immune system related disorder in a mammalian subject, wherein the composition is for administration to a lactating and postpartum female who is breast-feeding the mammalian subject.

According to another aspect, the present invention provides a method of enhancing and/or preventing an immune system related disorder in a mammalian subject, the method comprising providing or administering a composition comprising a non-digestible oligosaccharide to a lactating and postpartum female who is breast-feeding the mammalian subject.

According to yet another aspect, the present invention provides a method of providing or administering a composition comprising a non-digestible oligosaccharide to a lactating, postpartum female who is breast-feeding a mammalian subject, wherein the composition enhances an immune system and/or prevents an immune system related disorder in the mammalian subject.

For the purposes of the present invention, the oligosaccharide may be selected from the group consisting of galacto-oligosaccharides, fructo-oligosaccharides and acidic oligosaccharides. Suitably, the oligosaccharides used according to any aspect of the present invention may comprise, consists of, or consists substantially of galacto-oligosaccharide, fructo-oligosaccharide and acidic oligosaccharide.

Suitably, the galacto-oligosaccharide is a shortchain-galacto-oligosaccharide and/or the fructo-oligosaccharide is a longchain-fructo-oligosaccharide. Advantageously, a mammalian subject whose mother receives the non-digestible oligosaccharides during postpartum lactation (*e.g.* while breast-feeding the mammalian subject) may have a significantly reduced allergic response (*e.g.* undesirable allergic response) as compared to a mammalian subject whose mother did not receive the non-digestible oligosaccharide during postpartum lactation (*e.g.* while breast-feeding the mammalian subject). Suitably, the mammalian subject is a human.

### Non-digestible oligosaccharides

The non-digestible oligosaccharide may be a prebiotic oligosaccharide. These non-digestible oligosaccharides may beneficially affect the host by selectively stimulating the growth and/or activity of one or a limited number of bacterial species in the colon.

Suitably, the present non-digestible oligosaccharide may be soluble. Soluble oligosaccharide allow for ease of use and easy absorption into the body of the female.

The oligosaccharide may be a neutral oligosaccharide or an acidic oligosaccharide.

### Neutral non-digestible oligosaccharides

The phrase "neutral oligosaccharides" as used in the present invention refers to saccharides which have a degree of polymerisation of monose units exceeding 2, more suitably exceeding 3, even more suitably exceeding 4, most suitably exceeding 10, which are not or only partially digested in the intestine by the action of acids or digestive enzymes present in the human upper digestive tract (small intestine and stomach) but which are fermented by the human intestinal flora and preferably lack acidic groups. The neutral oligosaccharide may be structurally (chemically) different from an acid oligosaccharide.

The term neutral oligosaccharides as used in the present invention refers to saccharides which have a degree of polymerisation of the oligosaccharide below 100 monose units, suitably below 50, even more suitably below 25, most suitably below 10.

The term "monose units" refers to units having a closed ring structure, preferably hexose, e.g. the pyranose or furanose forms.

The neutral oligosaccharide may comprise at least 90%, more suitably at least 95% monose units selected from the group consisting of mannose, arabinose, fructose, fucose, rhamnose, galactose, β-D-galactopyranose, ribose, glucose, xylose and derivatives thereof, calculated on the total number of monose units contained therein.

Suitable neutral oligosaccharides may be fermented by the gut flora. Suitably, the oligosaccharide is selected from the group consisting of: cellobiose (4-O-β-D-glucopyranosyl-D-glucose), cellodextrins ((4-O-β-D-glucopyranosyl)ₙ-D-glucose), B-cyclodextrins (Cyclic molecules of α-1-4-linked D-glucose; α-cyclodextrin-hexamer, β-cyclodextrin-heptamer and γ-cyclodextrin-octamer), indigestible dextrin, gentiooligosaccharides (mixture of β-1-6 linked glucose residues, some 1-4 linkages), glucooligosaccharides (mixture of α-D-glucose), isomaltooligosaccharides (linear α-1-6 linked glucose residues with some 1-4 linkages), isomaltose (6-O-α-D-glucopyranosyl-D-glucose); isomaltriose (6-O-α-D-glucopyranosyl-(1-6)-α-D-glucopyranosyl-D-glucose), panose (6-O-α-D-glucopyranosyl-(1-6)-α-D-glucopyranosyl-(1-4)-D-glucose), leucrose (5-O-α-D-glucopyranosyl-D-fructopyranoside), palatinose or isomaltulose (6-O-α-D-glucopyranosyl-D-fructose), theanderose (O-α-D-glucopyranosyl-(1-6)-O-α-D-glucopyranosyl-(1-2)-B-D-fructofuranoside), D-agatose, D-/yxo-hexulose, lactosucrose (O-β-D-galactopyranosyl-(1-4)-O-α-D-glucopyranosyl-(1-2)-β-D-fructofuranoside), α-galactooligosaccharides including raffinose, stachyose and other soy oligosaccharides (O-α-D-galactopyranosyl-(1-6)-α-D-glucopyranosyl-β-D-fructofuranoside), β-galactooligosaccharides or transgalacto-oligosaccharides (β-D-galactopyranosyl-(1-6)-[β-D-glucopyranosyl]n-(1-4) α-D glucose), lactulose (4-O-β-D-galactopyranosyl-D-fructose), 4'-galatosyllactose (O-D-galactopyranosyl-(1-4)-0-P-D-glucopyranosyl-(1-4)-D-glucopyranose), synthetic galactooligosaccharide (neogalactobiose, isogalactobiose, galsucrose, isolactose I, II and III), fructans - Levan-type (β-D-(2→6)-fructofuranosyl)ₙ α-D-glucopyranoside), fructans - Inulin-type(P-D-((2→1)-fructofuranosyl)ₙ α-D-glucopyranoside), 1 f-β-fructofuranosylnystose (β-D-((2→1)- fructofuranosyl)ₙ B-D-fructofuranoside), xylooligosaccharides (B-D-((I→4)-xylose)ₙ, lafinose, lactosucrose and arabinooligosaccharides.

The non-digestible oligosaccharide according to any aspect of the present invention may be selected from a non-limiting group consisting of galacto-oligosaccharides (including but not limited to shortchain and longchain galacto-oligosaccharides), non-digestible dextrins (e.g. non-digestible polydextrins), xylo-oligosaccharides, arabino-oligosaccharides, gluco-oligosaccharides (including but not limited to geritiooligosaccharides and cyclodextrins), chito-oligosaccharides, fuco-oligosaccarides, manno-oligosaccharides, isomalto-oligosaccharides, glucomanno-oligosaccharides, galactomanno-oligosaccharides, arabinogalacto-olgosaccharides, fructo-oligosaccharides (including but not limited to longchain-fructo-oligosaccharides and shortchain-fructo-oligosaccharides), and the like.

In exemplary embodiments, the compositions described herein comprise, consists substantially of, consists of a shortchain-galacto-oligosaccharide, a longchain-fructo-oligosaccharide and an acidic-oligosaccharide. The phrase "consists substantially of" means that the composition includes the shortchain-galacto-oligosaccharide, the longchain-fructo-oligosaccharide and the acidic-oligosaccharide, and may also include one or more other elements, provided those elements do not interfere with or contributes to the activity or action of the shortchain-galacto-oligosaccharide, the longchain-fructo-oligosaccharide and the acidic-oligosaccharide. Thus, the shortchain-galacto-oligosaccharide, the longchain-fructo-oligosaccharide and the acidic-oligosaccharide are mandatory elements, and other elements are optional and may or may not be present. The phrase "consists of" means that the composition includes, and is limited to, the shortchain-galacto-oligosaccharide, the longchain-fructo-oligosaccharide and the acidic-oligosaccharide. Thus, the phrase "consists of indicates that the shortchain-galacto-oligosaccharide, the longchain-fructo-oligosaccharide and the acidic-oligosaccharide are mandatory elements, and that no other elements (such as other oligosacchairdes) may be present.

Galacto-oligosaccharide may be a non-digestible oligosaccharide, where at least 30% of the saccharide units are galactose units, suitably at least 50%, more suitably at least 60%. The galacto-oligosaccharide may have a DP of 2 to 100 or a DP of 2 to 10. In one non-limiting example, the saccharides of the galacto-oligosaccharide may be β-linked, as is the case in human milk oligosaccharide-core structures.

Suitably, the galacto-oligosaccharide may be selected from a non-limiting group consisting of transgalacto-oligosaccharides, lacto-N-tetraose (LNT) and lacto-N-neotetraose (neo-LNT). For example, the galacto-oligosaccharidemay comprise transgalacto-oligosaccharide ([galactose]n-glucose; wherein n may be an integer between 1 and 60, i.e. 2, 3, 4, 5, 6, 10, 20, 25, 30, 35, 40, 45, 43, 50, 56, 59, 60; most suitably n may be 2, 3, 4, 5, 6, 7, 8, 9 or 10). Transgalacto-oligosaccharides (TOS) are for example sold under the trademark Vivinal™ (Borculo Domo Ingredients, Netherlands). The saccharides of the galacto-oligosaccharides may be β-linked.

Fructo-oligosaccharide is a carbohydrate comprising a chain of at least 3 β-linked fructose units, with a DP between of 2 to 250, suitably between 7 to 100, more suitably between 20 to 60. For example, inulin may be used. Inulin is available under the tradename "Raftilin HP®", (Orafti). The average DP of the fructo-oligosaccharide may be at least 7, at least 10 or below 100. The inulin used may have the fructose units linked with a β(2→1) linkage. Fructo-oligosaccharides may include non-limiting compounds such as inulin, fructopolysaccharide, polyfructose, fructans, oligofructose and the like. The fructo-oligosaccharides may have a DP of 2 to 100.

Non-digestible polydextrins may refer to digestion-resistant (malto)dextrins or digestion-resistant polydextrose which may have a DP of 10 to 50, suitably between 10 and 20. The non-digestible polydextrins may comprise α(1→4), α(1→6) glucosidic bonds and 1→2 and 1→3 linkages. Non-digestible polydextrins may for example be available under the tradename Fibersol 2® from Matsutami Industries or Litesse® from Danisco.

Suitably, the non-digestible oligosaccharides may comprise an oligosaccharide with a degree of polymerization (DP) of 2 to 250. More suitably a DP of 2 to 100, even more suitably a DP 2 to 60, or a DP of 2 to 10.

The neutral oligosaccharide may be administered in an amount of between 10 mg and 100 gram per day, suitably between 100 mg and 50 grams per day, even more suitably between 0.5 and 20 gram per day.

Suitably, the compositions described herein may comprise neutral non-digestible oligosaccharides, wherein the non-digestible oligosaccharides are shortchain-galacto-oligosaccharides and longchain-fructo-oligosaccharides only. The weight ratio of shortchain-galacto-oligosaccharides and longchain-fructo-oligosaccharides may be about (20 to 2):1, for example, 20:1, 19:1, 18:1, 17:1, 16:1, 15:1, 14:1, 13:1, 12:1, 11:1, 10:1, 9:1, 8:1,7:1,6:1,5:1,4:1, 3:1 and 2:1.

### Acidic non-digestible oligosaccharides

The compositions described herein may comprise a non-digestible acidic-oligosaccharide. The term "acidic-oligosaccharide" refers to an oligosaccharide comprising at least one acidic group selected from the group consisting of N-acetylneuraminic acid, N-glycoloyneuraminic acid, free or esterified carboxylic acid, sulphuric acid group, and phosphoric acid group. The acidic-oligosacchide is suitably a polyhexose. In some embodiments, at least one of the aforementioned groups is situated at the terminal hexose unit of the acidic-oligosaccharide.

In exemplary embodiments, the acidic-oligosaccharide has the following structure: 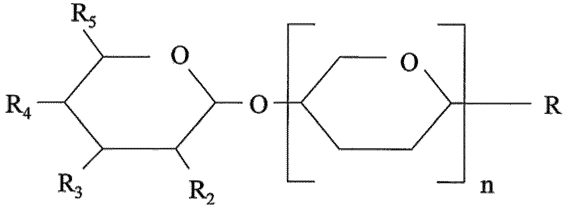 wherein the terminal hexose (left) suitably comprises a double bond. The acidic-oligosaccharide may contain a carboxylic acid at the terminal hexose unit, wherein said carboxylic acid group may be free or esterified. The hexose units other than the terminal hexose unit(s) are suitably uronic acid units, more suitably galaturonic acid units. The carboxylic acid groups on these units may be free or (partly) esterified, and suitably at least 10% is methylated (see below).

In some exemplary embodiments, the acidic-oligosaccharide used in the present invention comprises the polymeric acidic-oligosaccharide shown in the structure above, wherein:
R is selected from the group consisting of hydrogen, hyroxy or acid group, most suitably hydroxy; and
at least one selected from the group consisting of R₂, R₃, R₄ and R₅ represents N-acetylneuraminic acid, N-glycoloylneuraminic acid, free or esterified carboxylic acid, sulphuric acid group and phosphoric acid group, and the remaining of R₂, R₃, R₄ and R₅ represent hydroxy and/or hydrogen. In some embodiments, one of R₂, R₃, R₄ and R₅ represents free or esterified carboxylic acid, and the remaining of R₂, R₃, R₄ and R₅ represents hydroxy and/or hydrogen; and
n is an integer and refers to a number of hexose units (see also Degree of Polymerisation below), which may be any hexose unit. Suitably n is an integer between 1-5000.

In some embodiments, the hexose unit(s) is/are a uronic acid unit.

In one embodiment, R₁, R₂, and R₃ represent hydroxy, R₄ represents hydrogen, R₅ represents carboxylic acid, n is any number between 1 and 250, suitably between 1 and 10, and the hexose unit is galacturonic acid.

In some embodiments, the acidic-oligosaccharide has 1 or 2 terminal uronic acid units, which may be free or esterified. The terminal uronic acid unit may be selected from the group consisting of galacturonic acid, glucuronic acid, guluronic acid, iduronic acid, mannuronic acid, riburonic acid, and alturonic acid. These units may be free or esterified. In exemplary embodiments, the terminal hexose unit has a double bond, which may be situated between the C₄ and C₅ position of the terminal hexose unit. Suitably one of the terminal hexose units comprises the double bond. The terminal (e.g. uronic acid) suitably has a structure as follows: 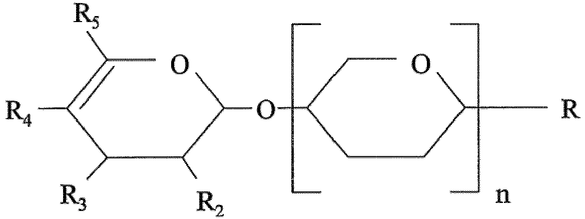 wherein;
R may be selected from the group consisting of hydrogen, hydroxy or acid group, suitably hydroxy (see above); and
at least one selected from the group consisting of R₂, R₃, R₄ and R₅ represents N-acetylneuraminic acid, N-glycoloylneuraminic acid, free or esterified carboxylic acid, sulphuric acid group and phosphoric acid group, and the remaining of R₂, R₃, R₄ and R₅ represent hydroxy and/or hydrogen. In some embodiments, one of R₂, R₃, R₄ and R₅ represents free or esterified carboxylic acid, and the remaining of R₂, R₃, R₄ and R₅ represents hydroxy and/or hydrogen; and
n is an integer and refers to a number of hexose units (see also Degree of Polymerisation below), which may be any hexose unit. Suitably n is an integer between 1-5000. In some embodiments, the hexose unit(s) is/are a uronic acid unit, suitably galacturonic acid units. The carboxylic acid groups on these units may be free or (partly) esterified, and may be at least partly methylated.

In one embodiment, R₂, and R₃ represent hydroxy, R₄ represents hydrogen, R₅ represents free or esterified carboxylic acid.

The compositions described herein may comprise more than one acidic-oligosaccharide, where each acidic-oligosaccharide has a different DP. Optionally, the compositions comprise an acidic-oligosaccharide comprising an unsaturated terminal hexose unit and an acidic-oligosaccharide comprising a saturated terminal hexose unit. In some embodiments, at least 5%, more suitably at least 10%, even more suitably at least 25% of the terminal hexose units of the acidic-oligosaccharides comprise unsaturated hexose units. As each individual acidic-oligosaccharide suitably comprises only one unsaturated terminal hexose unit, suitably no more than 50% of the terminal hexose units is an unsaturated hexose unit (i.e. comprises a double bond). In some embodiments, the compositions described herein comprise more than one acidic-oligosaccharide, comprising between 2 and 50% unsaturated hexose units based on the total amount of hexose units, suitably between 10 and 40%.

The acidic-oligosaccharide that may be used in the compositions described herein may have a DP between 1 and 5000, between 1 and 1000, between 2 and 250, between 2 and 50 or between 2 and 10. If a mixture of acidic-oligosaccharides with different degrees of polymerisation is used, the average DP of the acidic-oligosaccharide mixture may be between 2 and 1000, between 3 and 250 or between 3 and 50. A lower DP of the oligosaccharides may improve the palatability and results in a reduced viscosity product if the acidic-oligosaccharide is administered in liquid form. The acidic-oligosaccharide may comprise a homogeneous or a heterogeneous carbohydrate.

The acidic-oligosaccharides as used in the compositions described herein may be prepared from pectin, pectate, alginate, chondroitin, hyaluronic acids, heparin, heparan, bacterial carbohydrates, polygalacturonic acid and/or sialoglycans. Suitably, the acidic-oligosaccharide may be a pectin degradation product and/or alginate degradation product. Methods for the manufacture of esterified pectin hydrolysates that can be suitably used to prepare an acidic-oligosaccharide for use in the present invention are provided in WO 2001/60378 and/or WO 2002/42484, each of which is incorporated herein by reference. The detection, measurement and analyses of an acidic-oligosaccharide suitable for use in the present invention are provided in WO 2001/60378, the entire contents of which are incorporated herein by reference.

Alginates are linear unbranched polymers containing β-(1→4)-linked D-mannuronic acid and α-(1→4)-linked L-guluronic acid residues with a wide range of average molecular weights (100 - 100000 residues). Suitable sources of alginate include seaweeds and bacterial alginates.

Pectin may be divided into two main categories: high methoxylated pectin, which is characterised by a degree of methoxylation above 50% and low methoxylated pectin having a degree of methoxylation below 50%. The acidic-oligosaccharide described herein may be prepared from high methoxylated pectin or hydrolysed pectin. In some embodiments, the acidic-oligosaccharides used in the compositions of the present invention may be characterised by a degree of methoxylation above 20%, more suitably above 50%, even more suitably above 70%.

Suitably, the pectin degradation product may a pectin hydrolysate and or hydrolysed pectin (prepared by hydrolysis) and/or pectin lysate (prepared by beta-elimination). The pectin degradation product may be prepared from fruit or vegetable, for example, from apple pectin, citrus pectin, sugar beet pectin, and the like. The pectin degradation product may be prepared by lyases and/or variations of the temperature and/or pressure.

The acidic-oligosaccharide may be administered in an amount of between 10 mg and 100 gram per day, between 100 mg and 50 grams per day or between 0.5 and 20 gram per day.

The acidic-oligosaccharide may be included in the compositions described herein in an amount exceeding 0.1 wt.%, exceeding 0.2 wt.%, exceeding 0.5 wt.% or exceeding 1 wt.% based on the total dry weight of the composition. Although the administration of considerable amounts of oligosaccharides will generally not lead to undesirable side effects, the compositions described herein suitably have an oligosaccharide content below 20 wt.%, more suitably below 10 wt.%, most suitably below 5 wt.%.

### Nutritional compositions

The compositions described herein may be suitable for providing part or the full daily nutritional requirements to a lactating postpartum female. Thus, the composition may be used for feeding a lactating postpartum female, and may be a nutritional composition. The composition may therefore be formulated for enteral administration, including by oral administration. The composition is suitably not human breast milk.

The compositions described herein may comprise, consists essentially of, or consists of a shortchain-galacto-oligosaccharide, a longchain-fructo-oligosaccharide and an acidic-oligosaccharide. The acid- and neutral oligosaccharides may have a synergistic immune stimulatory effect.

The weight ratio of the shortchain-galacto-oligosaccharide, the longchain-fructo-oligosaccharide and the acidic-oligosaccharide may be about (20 to 2):1:(1 to 3), for example, 20:1:1, 19:1:1, 18:1:1, 17:1:1, 16:1:1, 15:1:1, 14:1:1, 13:1:1, 12:1:1, 11:1:1, 10:1:1, 9:1:1, 8:1:1, 7:1:1, 6:1:1, 5:1:1, 4:1:1, 3:1:1, 2:1:1, 20:1:2, 19:1:2, 18:1:2, 17:1:2, 16:1:2, 15:1:2, 14:1:2, 13:1:2, 12:1:2, 11:1:2, 10:1:2, 9:1:2, 8:1:2, 7:1:2, 6:1:2, 5:1:2, 4:1:2, 3:1:2, 2:1:2, 20:1:3, 19:1:3, 18:1:3, 17:1:3, 16:1:3, 15:1:3, 14:1:3, 13:1:3, 12:1:3, 11:1:3, 10:1:3, 9:1:3, 8:1:3, 7:1:3, 6:1:3, 5:1:3, 4:1:3, 3:1:3 and 2:1:3.

The compositions described herein may be formulated to provide 0.1 to 500 grams of the non-digestible oligosaccharides (e.g. per day), suitably between 0.5 to 100 grams of the further non-digestible oligosaccharides (e.g. per day). The compositions may be formulated to provide this daily dose in one portion per day, or this daily dose may be divided over 2 or 3 or 4 portions, which are intended to be consumed 2, 3 or 4 times per day.

The non-digestible oligosaccharide may be included in the compositions described herein in an amount exceeding 0.1 wt.%, exceeding 0.2 wt.%, exceeding 0.5 wt.% or exceeding 1 wt.% based on the total dry weight of the composition. Although the administration of considerable amounts of non-digestible oligosaccharides will generally not lead to undesirable side effects, the compositions may have a further non-digestible oligosaccharide content below 30 wt.%, suitably below 20 wt.% or more suitably 10 wt.%. In some embodiments, the further non-digestible oligosaccharide may be included in the composition in the range of between 0.1 wt% to 30 wt%, between 0.1 wt% to 20 wt%, or between 0.1 to 10 wt%.

The compositions described herein may comprise a lipid component, a protein component, and a digestible carbohydrate component.

In some embodiments, the lipid component used in the compositions described herein may comprise omega-3 (referred to as ω-3, n3 or n-3) fatty acids. In some embodiments, the lipid component may comprise omega-6 (referred to as ω-6, n6 or n-6) fatty acids. Nutritionally important omega-3 fatty acids include alpha-linolenic acid (ALA, 18:3n3), eicosapentaenoic acid (EPA, 20:5n3) and docosahexaenoic acid (DHA, 22:6n3). Nutritionally important omega-6 fatty acids include linoleic acid (LA, 18:2n6). Linoleic acid and alpha-linolenic acid are polyunsaturated essential fatty acids (PUFAs) as they cannot be synthesised in the human body, they must be sourced from the diet. They form the starting point for the creation of longer and more unsaturated fatty acids, which are also referred to as long-chain polyunsaturated fatty acids (LC-PUFAs) comprising at least 20 carbon atoms in the fatty acid chain and with 2 or more unsaturated bonds. The omega-3 ALA can be converted into EPA (20:5n3) which can be further converted into DPA (docosapentanenoic acid, 22:5n3), and further into DHA 30 (22:6n3). The omega-6 LA can be converted into arachidonic acid (AA, 20:4n6, an omega-6 fatty acid). The lipid component may comprise PUFAs and/or LC-PUFAs as free fatty free fatty acids, in triglyceride form, in diglyceride form, in monoglyceride form, in phospholipid form, or as a mixture of one of more of the above.

In some embodiments, the lipid component comprises at least one, suitably at least two lipid sources selected from the group consisting of linseed oil (flaxseed oil), rape seed oil (including colza oil, low erucic acid rape seed oil and canola oil), salvia oil, perilla oil, purslane oil, lingonberry oil, sea buckthorn oil, hemp oil, high oleic sunflower oil, high oleic safflower oil, olive oil, marine oils, microbial oils, black currant seed oil, echium oil, butter fat, butter oil, coconut oil and palm kernel oil.

In some embodiments, the protein component used in the compositions described herein may be selected from the group consisting of intact proteins including those sourced from animals (e.g. milk proteins) and vegetables (e.g. soy protein, pea protein and/or rice protein), hydrolysates of these proteins, peptides, free amino acids, and mixtures thereof.

The protein component may comprise casein, whey, hydrolysed casein and/or hydrolyzed whey protein. The protein component may comprise sweet whey with a reduced concentration of glycomacropeptide and/or acid whey. In some embodiments the protein component comprises protein derived from β-casein and/or α-lactalbumin. Suitably, the protein component comprises casein and whey proteins in a weight ratio casein:whey of 10:90 to 90:10, more suitably 20:80 to 80:20.

In some embodiments, the digestible carbohydrate component used in the compositions described herein may suitably comprise lactose, glucose, sucrose, fructose, galactose, maltose, starch or maltodextrin. Lactose is the main digestible carbohydrate present in human milk. The nutritional composition suitably comprises lactose.

The compositions described herein may further comprise components such as vitamins, minerals, trace elements and other micronutrients suitable for administration to a pregnant and/or lactating female. Often, supplements comprising vitamins and minerals are ingested by pregnant or lactating female for their or their infant's benefit. Inclusion of these vitamins and minerals in these compositions conveniently reduces the number of compositions (e.g. supplements) to be taken by pregnant and/or lactating female. Suitably, the composition therefore comprises at least one vitamin or mineral component, more suitably at least three vitamin an/or mineral components, even more suitably at least six vitamin and/or mineral components selected from the group consisting of folic acid, vitamin B1, vitamin B2, vitamin B6, vitamin A, vitamin D, iron, zinc, and iodine. In some embodiments, these components are present in a dosage that provides 25 to 100% of the recommended daily allowance (RDA), even more suitably between 45% and 100% RDA. The vitamin A may be supplied as β-carotene.

In some embodiments, the compositions described herein comprise a non-digestible oligosaccharide component, and the composition further comprises a lipid component, a protein component and a digestible carbohydrate component, wherein the non-digestible oligosaccharide component provides 0 to 10% of the total calories, the lipid component provides 10 to 70% of the total calories, the protein component provides to 10 to 70% of the total calories and the digestible carbohydrate component provides 30 to 60% of the total calories. In exemplary embodiments, the composition comprises a non-digestible oligosaccharide component, wherein the non-digestible oligosaccharide component provides 0 to 5% of the total calories, and wherein the composition further comprises a lipid component providing 20 to 60% of the total calories, a protein component provides 10 to 50% of the total calories and a digestible carbohydrate component provides 30 to 70% of the total calories. The amount of total calories may be determined by the sum of calories derived from non-digestible oligosaccharides, protein, lipids and digestible carbohydrates. In exemplary embodiments, the non-digestible oligosaccharide component may provide 1 to 4%, suitably 2 to 4% of the total calories, the lipid component may provide 20 to 45%, suitably 30 to 40% of the total calories, the protein component may provide 15 to 45%, suitably 20 to 35% of the total calories, and the digestible carbohydrate component may provide 40 to 60%, suitably 45 to 55% of the total calories.

In some other embodiments, the composition may comprise 50 to 200 kcal/100 ml liquid, more suitably 60 to 90 kcal/100 ml liquid, even more suitably 60 to 75 kcal/100 ml liquid. The caloric density can be formulated to ensure an optimal ratio between water and calorie consumption. The osmolarity of the present composition is suitably between 150 and 420 mOsmol/l, more suitably 260 to 320 mOsmol/l. A low osmolarity can be employed with the aim of reducing the gastrointestinal stress.

The composition may be in the form of a liquid (including a milk-based liquid), a powder, a bar, a capsule, or a tablet.

In some embodiments, the composition is in a liquid form. Suitably, the liquid composition has a viscosity below 35 mPa.s, even more suitably below 6 mPa.s as measured in a Brookfield viscometer at 20°C at a shear rate of 100 s⁻¹. When the composition is in a liquid form, it may be formulated for administration on a daily basis in the range of about 80 to about 2500 ml, more suitably about 200 to about 20 1200 ml per day. Suitably, the number of feedings per day is between 1 and 10, more suitably between 3 and 8.

The composition may be a powder suitable for making a liquid composition after reconstitution with an aqueous solution, such as water. Suitably, the composition may a powder to be reconstituted with water. For example, the powder may be packed in a sachet comprising 1 to 10 g, more suitably 1.5 to 7 g, most suitably 2 to 5 g.

In another example, the nutritional composition may be a milk-based liquid wherein the lipid component comprises less than 2 g/l in order to keep the amount of calories to be consumed low. The milk-based liquid may be packed into a bottle or tetrapack with a volume of 50 to 1000 ml, suitably 60 to 500 ml, more suitably 75 to 125 ml.

In another example, the composition may be a bar, for example a solid, chewable composition with a water activity below 0.8, suitably below 0.65.

The composition may be provided as a packaged powder or packaged ready-to-feed formula. To prevent spoilage of the product, packaging size of ready-to-feed formula suitably does not exceed one serving, e.g. does not exceed 500 ml; and packaging size of the composition may be in powder form and does not exceed 250 servings. Suitable packaging sizes for the powder are 2000 grams or less, more suitably per 1000 grams or less.

The composition may be formulated for administration on a regular basis (e.g. daily) for a period of at least 4 weeks, more suitably for a period of at least 8 weeks, most suitably for a period of at least 12 weeks, wherein the total volume administered daily may be between 200 ml and 1200 ml when the composition is in liquid form, and wherein the number of administrations per day may be between 1 and 10.

In some embodiments, the composition is for administration to a postpartum female who is breast-feeding her infant. Suitably, the composition is formulated for administration in the first 4 weeks post-partum (i.e. in the first 4 weeks of life of the infant), in the first 8 weeks post-partum, in the first 12 weeks post-partum, in the first 16 weeks post-partum, in the first 20 weeks post-partum, in the first 24 weeks postpartum, or any period in between. For example, the composition may be formulated for administration to the female in the weeks from 1, 2, 3, 4, 5, 6, 7, 8, 9, 10, 11, 12, 14, 16, 20, 24, 28, 32, or 36 weeks post-partum (or any integer in between) to weeks 2, 3, 4, 5, 6, 7, 8, 9, 10, 11, 12, 16, 20, 24, 28, 32, or 36 post-partum (or any integer in between). In some embodiments, the composition is formulated for administration to the female wherein the mammalian subject is of an age below 36 months, below 24 months, below 12 months, below 6 months, or any period in between. The female may exclusively breast-feed her infant or may breast-feed her infant and also feed her infant with infant formula, as it is recognized that there may be medical or personal reasons for a female to choose to feed her infant using a combination of her breast-milk and infant formula.

The present invention also contemplates packages comprising the compositions described herein along with a text (e.g. label) stating that upon consumption of the composition a lactating postpartum female will give her child an enhanced immune system, suitably including an enhanced immune system later in life, as further described herein. For example, the label may contain wording such as "stimulates maturation of the immune system", "reduces allergic reaction", "less stress", "reduced sensitivity" or similar wording.

### Applications

The present composition may be advantageously used to enhance an immune system, including an improved allergic response.

In some embodiments, the enhanced immune system comprises an improved allergic response in the mammalian subject. In exemplary embodiments, the immune system related disorder is a disorder or disease related to an allergic response including but not limited to atopic dermatitis, asthma, occupational asthma, food allergy (including hen's egg allergy, cow's milk allergy and others), allergic rhinitis (e.g. pollen allergy), dust mite allergy and other forms of hypersensitivity like systemic anaphylaxis and acute urticaria.

In some embodiments, the compositions described herein can be used to enhance an immune response and/or prevent an immune system related disorder in the mammalian subject, wherein at least the mother of the mammalian subject has an immune system related disorder. Thus in some embodiments, the female suffers from an immune system related disorder, or suffered from an immune system related disorder earlier in her life (e.g. during infancy or childhood).

The present invention is based on the discovery that administration of a composition comprising a non-digestible acidic-oligosaccharide as described herein to a female breast-feeding an infant has a long-term impact on the development and enhancement of the immune system in the infant.

The inventors have recognised this unexpected long-term effect on the immune response. Accordingly, in some embodiments the present invention aims to enhance the immune system (or immune response) and/or prevent an immune system related disorder of a mammalian subject (*e.g.* a human infant), where such enhancement or prevention effect occurs in the mammalian subject at a time when the composition is no longer administered to the mother of the mammalian subject (*e.g.* the mammalian subject has been born or is no longer breast-feeding, for example a human infant who has weaned or is an adolescent), suitably exceeding the age by at least one year, suitably by 2 years, suitably by 3 years, more suitably by 5 years, most suitably by 8 years.

Thus in some exemplary embodiments, the compositions described herein may enhance the immune response and/or prevent an immune system related disorder later in life in the mammalian subject (*e.g.* human subject). With "later in life" is meant an age exceeding the age at which the mammalian subject is breast-fed, suitably exceeding the age by at least 12 months, more suitably by 24 months, by 36 months, by 5 years, most suitably by 8 years.

Having now generally described the invention, the same will be more readily understood through reference to the following examples which are provided by way of illustration, and are not intended to be limiting of the present invention.

A person skilled in the art will appreciate that the present invention may be practised without undue experimentation according to the method given herein. The methods, techniques and chemicals are as described in the references given or from standard protocols.

### EXAMPLES

### Example 1: Maternal supplementation with non-digestible oligosaccharides

### Materials & Methods

### Chemicals

Ovalbumin (Grade V) was obtained from Sigma (St. Louis, USA). Cholera toxin was purchased from Quadratech Diagnostics. Phosphate buffered saline (PBS) was obtained from BioWhittaker (Verviers, Belgium).

### Animals

3 week old female C3H/HeOuJ mice were obtained from Charles River (France) and housed at constant temperature (20°C) and humidity (40-60%) at a 12:12 hour light/dark cycle in the animal facility of Utrecht University. Animal care and use were performed in accordance with the guidelines of the Dutch Committee of Animal Experiments and the local university ethical committee.

### Experimental setup

Female mice were sensitised intragastric (i.g.) once a week for five weeks with hen's egg ovalbumin (OVA) (20 µg/500µl PBS) using cholera toxin (CT) (10 µg/500µl PBS) as adjuvant. Control groups received either phosphate-buffered saline (PBS) or PBS + CT. Two weeks after the last sensitisation mice were mated and fed either a control diet (AIN93) or the AIN93 diet supplemented with a specific non-digestible oligosaccharide mixture containing shortchain-galacto-, longchain-fructo- and pectin-derived acidic-oligosaccharides (1%, ratio 9:1:2) (GFA) during pregnancy or lactation (Table 1).

**Table 1. Description of the 9 groups of mice**

| Group | Treatment to dams | Maternal diet during pregnancy | Maternal diet during lactation |
|---|---|---|---|
| 1 (sham) | PBS | AIN93 (control) | AIN93 |
| 2 (control) | PBS | AIN93 | AIN93 |
| 3 (pregnancy) | PBS | GFA | AIN93 |
| 4 (control) | CT | AIN93 | AIN93 |
| 5 (pregnancy) | CT | GFA | AIN93 |
| 6 (lactation) | CT | AIN93 | GFA |
| 7 (control) | CT + OVA | AIN93 | AIN93 |
| 8 (pregnancy) | CT + OVA | GFA | AIN93 |
| 9 (lactation) | CT + OVA | AIN93 | GFA |

After weaning, all female offspring were transferred to the control diet and sensitised orally once a week for four weeks with OVA + CT. One week after the last sensitisation, the offspring were challenged intradermally with 20µg OVA/20µl PBS in the ear, with the exception of animals in group 1, which received PBS intradermally. Individual shock scores were assessed 30 minutes after intradermal challenge. In addition, the temperature of the mice was measured 1 minute before intradermal challenge, and every 15 minutes after challenge until the ear swelling (as a measure of the acute allergic skin response to OVA) was measured after one hour. Ear thickness was measured in duplicate before antigen challenge and 1 hour afterwards using a digital micrometer (Mitutoyo Digimatic 293561; Veenendaal, The Netherlands). The acute allergic skin response was calculated by subtracting the basal ear thickness from the value at 1 hour after challenge (Δ ear thickness), and values are given as ΔµM. Approximately 6 hours after the intradermal challenge, mice were challenged orally with OVA (100 mg/500µl). 18 hours after the oral challenge, mice were sacrificed and samples were collected for *ex-vivo* analyses.

### Plasma Antibody Concentrations

OVA-specific immunoglobulins were measured as described previously (Deurloo *et al*.). Values are given as arbitrary units (A.U.) as the standard curve was prepared from the pooled samples of all the animals in the experiment.

### Results

### Acute allergic skin response

The acute allergic skin response in the offspring of OVA-sensitised dams supplemented with the non-digestible oligosaccharides (scGOS/IcFOS/pAOS) during pregnancy or lactation was significantly (p < 0.05) reduced compared to offspring of OVA-sensitized mice fed the control diet during pregnancy or lactation (Figure 1). This effect was observed for both the offspring of OVA-sensitised dams supplemented during pregnancy as well as lactation. In contrast to these results, supplementation did not significantly attenuate the acute allergic skin response in the offspring of the non-sensitised dams (i.e. those who received PBS or PBS + CT prior to mating) (Figure 1).

### Shock scores and temperature

Shock scores as a result of the intradermal challenge with OVA were significantly higher in groups 2-7 when compared to the sham-challenged mice in group 1. The shock scores of mice from groups 8 and 9 did not differ significantly from the sham-challenged mice (Figure 2). This observation is in line with the results from the acute allergic skin response. However, as scoring shock symptoms may be somewhat subjective, the temperature of the mice was also measured (Figure 3). A decrease in temperature after intradermal challenge is a quantifiable shock symptom as a result of acute allergic response to OVA. Compared to the sham-challenged mice in group one, there was a significant decrease in temperature 30 minutes after intradermal challenge with OVA in groups 2, 3, 4 and 5. In groups 6-9, there was no significant decrease in temperature as compared to the sham-challenged animals.

### OVA-specific immunoglobulins

When comparing the OVA-specific IgE levels in offspring of OVA-sensitised, supplemented dams (group 8 and 9) to sensitised, non-supplemented dams (group 7), a decrease in OVA-specific IgE levels was only observed in the offspring of dams supplemented during lactation (Figure 4A). Similarly, no significant difference in OVA-specific IgE levels was observed between the offspring of non-supplemented PBS-treated dams (group 2) and those supplemented during pregnancy (group 3) (Figure 4A). In contrast, a significant decrease in OVA-specific IgE was observed both in offspring of CT-treated dams supplemented during pregnancy (group 5), and those supplemented during lactation (group 6) when compared to the offspring of non-supplemented dams (group 4) (Figure 4A). OVA-specific IgE levels in group 1 were only significantly different compared to group 4, however, as these mice were not challenged with OVA at the end of the experiment they cannot be compared to the other groups (data not shown).

OVA-specific IgG1 levels differed significantly between group 4 and 6, and group 7 and 9, indicating that supplementation during lactation can affect the production of this Th2-related immunoglobulin in the offspring regardless of the treatment of the dams (Figure 4B)

OVA-specific IgG2a levels were significantly higher in the offspring of CT-treated and OVA+CT-treated dams supplemented during pregnancy or lactation when compared to offspring of non-supplemented dams. This effect was not observed in offspring of PBS-treated dams (Figure 4C).

The results in Example 1 suggest that maternal supplementation with non-digestible carbohydrates has a beneficial programming effect on the immune response of offspring.

### Example 2: Liquid composition

A liquid milk-based composition packed in a 100 ml, 230 ml or 250 ml bottle, prepared with a skim milk, semi-skimmed milk, or whole-milk base, wherein the composition comprises (per 100 ml) 100, 120, 140, 149, or 160 kJ, including a non-digestible oligosaccharide component (0.5-30 g non-digestible oligosaccharide component), a protein component (typically 8-30 g protein), a digestible carbohydrate (typically 20-80 g digestible carbohydrate), and a lipid component (typically 0.2 - 20 g fat), a polyunsaturated fatty acid, minerals, trace elements and vitamins. In this composition, the non-digestible oligosaccharide includes a galacto-oligosaccharide (e.g. 8-10 g originating from Vivinal GOS) and a fructo-oligosaccharde (e.g. 0.5-1 g fructopolysaccharide originating from RaftilinelHP), and optionally an acidic-oligosaccharide, typically a uronic acidic-oligosaccharide.

The label on the package for this composition indicates that the composition is for a mother while pregnant and/or while breast-feeding her infant, and may also state that the composition enhanced the immune system of the infant later in life, for example, the label may contain wording such as "stimulates maturation of the immune system", "reduces allergic reaction", "less stress", "reduced sensitivity" or similar wording.

### Example 3: Liquid composition

A powder composition is prepared based on skim milk, semi-skimmed milk, or whole-milk base, wherein the composition comprises (per 100 g) 300, 350, 365, or 400 kJ, including a non-digestible oligosaccharide component (0.5-60 g non-digestible oligosaccharide component), a protein component (typically 8-30 g protein), a digestible carbohydrate (typically 20-80 g digestible carbohydrate), and a lipid component (typically 0.2 - 20 g fat), a polyunsaturated fatty acid, minerals, trace elements and vitamins. In this composition, the non-digestible oligosaccharide includes a galacto-oligosaccharide (e.g. 8-10 g originating from Vivinal GOS) and a fructo-oligosaccharde (e.g. 0.5-1 g fructopolysaccharide originating from RaftilinelHP), and optionally also includes an acidic-oligosaccharide, typically a uronic acidic-oligosaccharide,

The label on the package for this composition indicates that the composition is for a mother while pregnant and/or while breast-feeding her infant, and may also state that the composition enhanced the immune system of the infant later in life, for example, the label may contain wording such as "stimulates maturation of the immune system", "reduces allergic reaction", "less stress", "reduced sensitivity" or similar wording.

### REFERENCES

1. Deurloo DT, van Esch BC, Hofstra CL, Nijkamp FP, van Oosterhout AJ. CTLA4-IgG reverses asthma manifestations in a mild but not in a more "severe" ongoing murine model. Am J Respir Cell Mol Biol. 2001; 25: 751-60.
2. L. Prosky et al., J. Assoc. Off. Anal. Chem. 71, 1017-1023 (1988).
3. Lucas, A. 2000, "Programming not metabolic imprinting", Am. J. Clin. Nutr., 71:602.
4. WO 2002/42484.
5. WO 2001/60378.

## Claims

1. A composition comprising a non-digestible oligosaccharide for use in enhancing an immune system and/or preventing an immune system related disorder in a mammalian subject, wherein the composition is for administration to a lactating, postpartum female who is breast-feeding the mammalian subject.

2. The composition according to claim 1, wherein the non-digestible oligosaccharide is selected from the group consisting of galacto-oligosaccharide, fructo-oligosaccharide and acidic oligosaccharide.

3. The composition according to any one of the preceding claims, wherein the non-digestible oligosaccharide is galacto-oligosaccharide and fructo-oligosaccharide in a weight ratio of 9:1, 5:1 or 3:2.

4. The composition according to any one of the preceding claims, wherein the non-digestible oligosaccharide is galacto-oligosaccharide, fructo-oligosaccharide and acidic oligosaccharide in a weight ratio of 9:1:2, 5:1:2 or 7:1:3.

5. The composition according to any one of the preceding claims, wherein the composition comprises non-digestible oligosaccharides in a range of 0.1 wt% to 10 wt%.

6. The composition according to any one of the preceding claims, wherein the composition is for administration in a dose of 0.1 to 500 g/day.

7. The composition according to any of the preceding claims, wherein the mammalian subject is a human subject.

8. The composition according to claim 7, wherein the human subject has an age below 36 months.

9. The composition according to either claim 7 or 8, wherein the immune system of the human subject is enhanced when the human subject has an age above 12 months.

10. The composition according to any one of the preceding claims, wherein the immune system related disorder is an unwanted, undesirable and/or an excessive allergic reaction.

11. The composition according to any one of the preceding claims, wherein the female has an immune system related disorder.

## Patentansprüche

1. Zusammensetzung, umfassend ein nicht-verdauliches Oligosaccharid, zur Verwendung beim Stärken eines Immunsystems und/oder Verhindern einer mit dem Immunsystem zusammenhängenden Erkrankung bei einem Säugersubjekt, wobei die Zusammensetzung für Verabreichung an ein laktierendes, postpartales weibliches Wesen bestimmt ist, welches das Säugersubjekt stillt.

2. Zusammensetzung gemäß Anspruch 1, wobei das nicht-verdauliche Oligosaccharid aus der Gruppe, bestehend aus Galactooligosaccharid, Fructooligosaccharid und saurem Oligosaccharid, ausgewählt ist.

3. Zusammensetzung gemäß einem der vorhergehenden Ansprüche, wobei das nicht-verdauliche Oligosaccharid Galactooligosaccharid und Fructooligosaccharid in einem Gewichtsverhältnis von 9:1, 5:1 oder 3:2 ist.

4. Zusammensetzung gemäß einem der vorhergehenden Ansprüche, wobei das nicht-verdauliche Oligosaccharid Galactooligosaccharid, Fructooligosaccharid und saures Oligosaccharid in einem Gewichtsverhältnis von 9:1:2, 5:1:2 oder 7:1:3 ist.

5. Zusammensetzung gemäß einem der vorhergehenden Ansprüche, wobei die Zusammensetzung nicht-verdauliche Oligosaccharide in einem Bereich von 0,1 Gew.-% bis 10 Gew.-% umfasst.

6. Zusammensetzung gemäß einem der vorhergehenden Ansprüche, wobei die Zusammensetzung für Verabreichung in einer Dosis von 0,1 bis 500 g/Tag bestimmt ist.

7. Zusammensetzung gemäß einem der vorhergehenden Ansprüche, wobei das Säugersubjekt ein humanes Subjekt ist.

8. Zusammensetzung gemäß Anspruch 7, wobei das humane Subjekt ein Alter unter 36 Monaten hat.

9. Zusammensetzung gemäß entweder Anspruch 7 oder 8, wobei das Immunsystem des humanen Subjekts gestärkt wird, wenn das humane Subjekt ein Alter über 12 Monaten hat.

10. Zusammensetzung gemäß einem der vorhergehenden Ansprüche, wobei die mit dem Immunsystem zusammenhängende Erkrankung eine ungewünschte, nicht wünschenswerte und/oder eine übermäßige allergische Reaktion ist.

11. Zusammensetzung gemäß einem der vorhergehenden Ansprüche, wobei das weibliche Wesen eine mit dem Immunsystem zusammenhängende Erkrankung hat.

## Revendications

1. Composition comprenant un oligosaccharide non digestible destiné à être utilisé pour renforcer un système immunitaire et/ou prévenir un trouble du système immunitaire chez un sujet mammifère, laquelle composition est destinée à être administrée pendant la période postnatale à une femelle en lactation, qui allaite le sujet mammifère.

2. Composition selon la revendication 1, dans laquelle l'oligosaccharide non digestible est choisi dans le groupe constitué par un galacto-oligosaccharide, un fructo-oligosaccharide et un oligosaccharide acide.

3. Composition selon l'une quelconque des revendications précédentes, dans laquelle l'oligosaccharide non digestible consiste en galacto-oligosaccharide et fructo-oligosaccharide dans un rapport pondéral de 9:1, 5:1 ou 3:2.

4. Composition selon l'une quelconque des revendications précédentes, dans laquelle l'oligosaccharide non digestible consiste en galacto-oligosaccharide, fructo-oligosaccharide et oligosaccharide acide dans un rapport pondéral de 9:1:2, 5:1:2 ou 7:1:3.

5. Composition selon l'une quelconque des revendications précédentes, laquelle composition comprend des oligosaccharides non digestibles à raison de 0,1% en poids à 10% en poids.

6. Composition selon l'une quelconque des revendications précédentes, laquelle composition est destinée à être administrée à une dose de 0,1 à 500 g/jour.

7. Composition selon l'une quelconque des revendications précédentes, dans laquelle le sujet mammifère est un sujet humain.

8. Composition selon la revendication 7, dans laquelle le sujet humain est âgé de moins de 36 mois.

9. Composition selon la revendication 7 ou 8, dans laquelle le système immunitaire du sujet humain est renforcé lorsque le sujet humain est âgé de plus de 12 mois.

10. Composition selon l'une quelconque des revendications précédentes, dans laquelle le trouble du système immunitaire est une réaction allergique non souhaitée, indésirable et/ou excessive.

11. Composition selon l'une quelconque des revendications précédentes, dans laquelle la femelle présente un trouble du système immunitaire.
